# EUROPEAN PATENT SPECIFICATION

(11) **EP 4 463 106 B1**
(45) Date of publication and mention of the grant of the patent: **11.03.2026**
(21) Application number: 23703639.7
(22) Date of filing: 10.01.2023
(51) Int. Cl.: A61F 2/66

(54) **PROSTHETIC IMPLANT OF THE ESR TYPE AND METHOD FOR ADJUSTING THE PROSTHETIC IMPLANT**
EIN PROTHETISCHES IMPLANTAT VOM ENERGIESPEICHERNDEN UND ZURÜCKGEBENDEN TYP UND EIN VERFAHREN ZUM ANPASSEN EINES PROTHETISCHEN IMPLANTATS
IMPLANT PROTHETIQUE DU TYPE STOCKAGE ET RETOUR D'ENERGIE ET PROCEDE D'AJUSTEMENT D'UN IMPLANT PROTHETIQUE

(30) Priority: 13.01.2022 IT 202200000485
(43) Date of publication of application: 20.11.2024
(73) Proprietor: Alma Mater Studiorum Universita di Bologna, 40126 Bologna (IT)
(72) Inventor: TABUCOL, Johnnidel, 40061 MINERBIO (IT); LEOPALDI, Marco, 80016 Marano di Napoli (IT); BRUGO, Tommaso Maria, 40125 Bologna (IT); ZUCCHELLI, Andrea, 40069 Zola Predosa (BOLOGNA) (IT)
(74) Representative: Conti, Marco
(86) International application number: PCT/IB2023/050195
(87) International publication number: WO 2023/135510

(56) References cited:
- EP-A1- 3 128 958
- US-A1- 2020 375 764
- US-B2- 10 413 429

## Description

### Technical field

This invention relates to a prosthetic implant of the ESR (energy-storing-and-returning) type and to a method for adjusting a prosthetic implant of the ESR type which is widely used in the medical field.

In particular, this invention is widely used in the field of lower limb prostheses and, more specifically, in the field of foot prostheses.

### Background art

As is known, during walking, the foot undergoes a load cycle, depending mainly (but not only) on the conditions of the ground, the person's weight, a load being carried, if any, and the walking speed, to which the foot reacts by adapting its rigidity.

To date, therefore, there is a particularly strongly felt need to ensure that amputees have their ambulating functions restored almost entirely to the conditions existing prior to amputation. To meet this need, various different prosthetic implants are available as substitutes which aim to replicate as faithfully as possible the complexities of the force-deformation curve representing a patient's natural gait cycle.

In other words, the aim, to date, is to create a prosthetic implant that is capable of providing a mechanical reaction to different ground types, different obstacles and, generically, different loads, that is as close as possible to the mechanical reaction of a natural foot.

In this situation, various different prosthetic implants have been developed which, by varying the stiffness of the implant itself, allow the patient to walk not only on flat ground, but also on irregular surfaces such as, for example, footpaths, uneven ground, stairs, ramps and the like.

An example of such prosthetic implants is disclosed in US10034782B2. That patent document discloses an implant comprising an elongate prosthetic foot element in the form of a blade provided with a longitudinal slot. The implant also comprises a tongue portion configured to flex independently of the foot element. The prosthetic implant also includes a selectively actuated mechanism to operatively connect/disconnect the tongue portion to/from the foot element. More in detail, when the tongue portion is operatively connected to it, the foot element exhibits greater stiffness, and when the tongue portion is operatively disconnected from it, the foot element exhibits lower stiffness.

In other words, coupling and uncoupling the tongue portion and the foot element to and from each other allows varying the stiffness of the implant so as to adapt it to the patient's ambulation requirements. Disadvantageously, actuating the mechanism which connects/disconnects the tongue portion is particularly complex and difficult, especially if the mechanism needs to be actuated when the prosthetic implant is in use. Another disadvantage is due to the fact that in the prosthetic implant described in US10034782B2, the variable stiffness is limited to the foot element only. That means the implant is less flexible and less adaptable to the patient's ambulation requirements in that it does not allow varying and adjusting the torsional stiffness of the ankle portion of the implant.

This drawback is at least partly overcome by the prosthetic implant disclosed in US9289316B2. That patent document discloses an implant comprising a main body (or ankle) coupled to a foot by an articulated system. The articulated system acts as a variable lever arm for adapting the response of the implant to the patient's movement.

In particular, the articulated system comprises a spring having one end hinged to the main body at a fixed hinge point and another end engaged with the foot by a mounting block that is slidable along a horizontal direction (parallel to the patient's direction of movement).

When the patient walks, the sliding of the mounting block causes a variation of the length of the lever arm defined by the articulated system. Adjusting the lever arm makes it possible to control the torsional stiffness of the articulated system. More specifically, the torsional stiffness is linked to the linear stiffness of the spring and to the length of the lever arm. Thus, changing the position of the mounting block has the effect of changing the torsional stiffness of the prosthetic implant.

Disadvantageously, since the torsional stiffness of the prosthetic implant depends on a spring with linear stiffness, the prosthetic implant of US9289316B2 is unable to precisely and reliably mimic the non-linear trend of the force-deformation curve representing the patient's natural gait cycle. This drawback is at least partly overcome by prosthetic implants whose torsional stiffness can be varied by a system of shock absorbers operating with thickening fluids which, by varying their viscosity, allow the prosthetic implant to respond adaptively.

In other words, depending on the phase of the load cycle the prosthetic implant is subjected to (that is, depending on which phase of their gait cycle the patient is in), the viscosity of the thickening fluids varies to allow the implant to react in an optimal manner.

Disadvantageously, these prosthetic implants also have drawbacks. In particular, as the patient walks, for the prosthetic implant to respond adequately to load variations due, for example, to stairs, uneven ground, obstacles and the like, the response reaction of the implant with the shock absorber system needs to be well calibrated. That means that the energy that might be accumulated in an unloading phase of the cycle (known as "stance phase") is lost and thus cannot be used by the patient in the step that follows (in what is known as the "push-off phase"). In this situation, prosthetic implants that exploit the viscosity variation of thickening fluids are uncomfortable to use and have a considerable fatiguing effect on the patient.

Other examples of foot prostheses are known from patent documents EP3128958A1 and US2020/375764A1. EP3128958A1 involves varying the length of the element that connects the top plate assembly to the blades. Even these solutions, however, do not allow the stiffness to be adjusted in a simple and precise manner.

Also known are fully actuated prostheses which can vary the stiffness but which require the actuating motor to work at each step the patient takes. This has an enormous impact on mobility.

The technical purpose of this invention, therefore, is to provide a prosthetic implant of the ESR (energy-storing-and-returning) type and a method for adjusting a prosthetic implant of the ESR type to overcome the above mentioned disadvantages of the prior art.

### Disclosure of the invention

The invention is defined in the claims. The aim of this invention, therefore, is to provide a prosthetic implant of the ESR (energy-storing-and-returning) type, but also of the semi-active ESR and active ESR type, capable of replicating as faithfully as possible the force-deformation curve representing the patient's natural gait cycle.

Another aim of this invention is, therefore, to provide a prosthetic implant of the ESR (energy-storing-and-returning) type that is comfortable for the user and reliable in use.

The technical purpose indicated and the aims specified are substantially achieved by a prosthetic implant of the ESR (energy-storing-and-returning) type and a method for adjusting a prosthetic implant of the ESR type, comprising the technical features set out in one or more of the appended claims. The dependent claims correspond to possible embodiments of the invention.

More specifically, the technical purpose claimed and the aims specified are achieved by a prosthetic implant of the ESR (energy-storing-and-returning) type. The prosthetic implant comprises an upper assembly configured to be connected to a patient's lower limb, and a lower assembly configured to define a foot.

The foot has a front region, a middle region and a rear region. The lower assembly includes a lower blade, extending along a main direction between a heel end and a toe end.

According to an aspect of the disclosure, the lower blade, along the main direction, has a curved shape that replicates the curvature of a sole of a foot.

According to another aspect of the disclosure, the lower blade is provided, along the main direction, with a longitudinal slot extending from the toe end substantially up to the middle region of the foot.

The slot divides the toe end into two rounded portions defining a "forefoot portion" (that is, the part defining the toes of the foot).

The lower assembly also comprises an intermediate blade that is partly superposed on the lower blade, preferably in the middle region of the foot. In the preferred embodiment, the intermediate blade has a first straight stretch extending from a toe end of the intermediate blade and at least partly superposed on the lower blade.

In the preferred embodiment, the intermediate blade also has a second straight stretch extending at an angle to the first stretch. The second stretch extends from the first stretch towards a heel end of the intermediate blade. According to an aspect of the disclosure, the intermediate blade also has a longitudinal slot, extending from the heel end to a middle zone of the intermediate blade itself.

The lower assembly also comprises an upper blade that is partly superposed on the intermediate blade, preferably in the middle region of the foot.

In the preferred embodiment, the upper blade is substantially rectilinear in shape.

The upper blade, the intermediate blade and the lower blade are superposed on each other substantially in the middle region of the foot. More precisely, the upper blade, the intermediate blade and the lower blade are superposed on each other between the middle region and the front region of the foot.

The upper blade, the intermediate blade and the lower blade are fastened stably to each other at at least one connecting point located in the front region or in the middle region of the foot.

In a possible embodiment, the connecting point comprises two parallel pins extending from the upper blade to the lower blade through the intermediate blade.

The upper, lower and intermediate blades diverge from each other away from the connecting point towards the rear region of the foot.

The upper, lower and intermediate blades are laminated springs made from composite material. In this situation, the upper, lower and intermediate blades have a non-linear elastic behaviour.

The prosthetic implant also comprises an articulated joint, interposed between the lower assembly and the upper one to allow relative motion between the lower assembly and the upper one.

The articulated joint acts as a ball joint allowing the patient to walk, that is to say, to mimic an ankle joint.

In a possible embodiment, the articulated joint includes a ball that is stably fixed to the upper assembly and faces towards the lower assembly. The articulated joint also includes a receiving body that is fixed to the lower assembly and faces towards the upper assembly to receive the ball. The receiving body is fixed to the upper blade in the proximity of its heel end.

In a possible embodiment, the receiving body is distinct from the lower assembly, and more specifically, from the upper blade, and is fastened to the lower assembly by, for example, screws or the like.

Alternatively, the receiving body is made as a single part with the upper blade.

The ball and the receiving body define a ball and socket joint. Alternatively, the ball and the receiving body define a universal joint or a simple hinge.

In an example, the ball and the receiving body are made from composite material. Alternatively, the ball and the receiving body may be made from a material customarily used to make prostheses.

The prosthetic implant also comprises a connector having a first end that is connected to the upper assembly, and a second end that is connected to the intermediate blade of the lower assembly to apply a force on the intermediate blade of the lower assembly.

The connector acts substantially as a tendon element configured for transmitting a force between the upper assembly and the lower assembly. In a possible embodiment, the connector comprises a first portion which extends from the first end and a second portion which extends from the second end and which is configured to be connected to the first portion. The first and the second portion define a lead nut and screw connection. According to an aspect of this disclosure, the connector is a rigid element of adjustable length.

According to a possible example embodiment, the length of the connector is adjustable manually. According to another embodiment, the length might be adjustable automatically by means of a motor.

According to another aspect of this disclosure, at least one between the first and the second end of the connector is movable along a movement direction to vary the force applied on the intermediate blade of the lower assembly. In this situation, it is possible to vary the stiffness of the foot by adapting it easily and precisely to the different loads the patient is subjected to and/or to the different types of ground the patient walks on. The connector is elongated along a connection axis. The movement direction is inclined with respect to the connection axis. The movement direction is also inclined with respect to the longitudinal direction. Preferably, the movement direction is perpendicular to the longitudinal direction. The inclination angle between the movement direction and the connection axis varies with the changing position of the movable end of the connector; for example, the angle might be ninety degrees (or slightly greater); more generally speaking and by way of example, the angle might vary between 70 (or 90) and 130 degrees. It should be noted that the change in position (displacement) of the at least one between the first and the second end of the connector along a movement direction does not, in itself, involve any change of length of the connector (the connector might even be of fixed length). Preferably, it is the first end of the connector that is movable along the movement direction (that is, along a movement axis).

More in detail, being able to control the change in the connector fastening position where the force is exchanged between the lower assembly (comprising the blades, which have non-linear behaviour) and the upper assembly makes it possible to vary the point where the force is applied. In this situation, the lever arm of the force relative to the centre of rotation of the ankle changes, hence the transmitted torque also changes. By so doing, the variation in the torsional stiffness obtained can be attributed to the entire foot and is a non-linear variation, that is, a variation that replicates the natural behaviour of a foot in a highly faithful manner.

In an embodiment, the upper assembly extends along a longitudinal direction between a hook-up end, where it is operatively connected to the patient's lower limb, and a fixing end, where it is fixed to the articulated joint. The first end of the connector is slidably movable relative to the upper assembly and the movement direction is transverse to the longitudinal axis. More specifically, the movement direction is perpendicular to the longitudinal axis.

In the preferred embodiment, when the first end of the connector is slidably movable relative to the upper assembly, the second end of the connector is articulated to the intermediate blade of the lower assembly.

More specifically, the second end of the connector is hinged to the intermediate blade of the lower assembly so that a sliding movement of the first end of the connector corresponds to a rotational movement of the connector about the second end.

According to an aspect of the disclosure, the prosthetic implant comprises a slider that is extractable or retractable relative to the upper assembly along the movement direction through a plurality of equilibrium positions.

The first end of the connector is pivoted to the slider to adjust the position of the first end itself between a most extracted position, where the first end is distal to the upper assembly, and a least extracted position, where the first end is proximal to the upper assembly.

At the most extracted position, the slider is extracted and the connector is rotated about the second end so that the first end is distal to the upper assembly. In this situation, the prosthetic implant exhibits a high value of stiffness.

At the least extracted position, the slider is retracted and the connector is rotated about the second end so that the first end is close to the upper assembly. In this situation, the prosthetic implant exhibits a lower value of stiffness.

The slider might also be partly extracted from the upper assembly to occupy an intermediate position between the most extracted position and the least extracted position. The intermediate position corresponds to a walking state of the patient.

Instead of the slider, the prosthetic implant might, for example, comprise a cam capable of varying the position of the first end of the connector as described above. In use, therefore, by controlling (by extracting or retracting the slider) the position of the fastening point (that is, of the first end) of the connector, where the force is exchanged between the lower assembly and the upper assembly, it is possible to vary the point where the force is applied. This also changes the lever arm of the force relative to the centre of rotation of the ankle (articulated joint), thus varying the transmitted torque, hence the torsional stiffness of the entire foot. Since the blades have a non-linear elastic behaviour, the variation in the stiffness obtained is non-linear and such as to better replicate the force-deformation curve of a natural foot subjected to different states of stress and/or loads (for example, uneven ground, weights lifted, and so on).

In the preferred embodiment, the slider is coupled to the upper assembly by a prismatic coupling.

Alternatively, the slider may be coupled to the upper assembly by any other coupling that allows translational motion in the desired direction and is constrained in the other translational and rotational movements.

In a possible embodiment, the first end is movable slidably via an actuator which is, for example, connected to the upper assembly.

Alternatively, the first end is movable slidably by manual operation.

In another possible embodiment, the first end of the connector may be articulated to the upper assembly - hinged to it, for example - while the second end may be movable slidably along the intermediate blade.

In this situation, the prosthetic implant comprises a slide that is slidably movable along the intermediate blade to steplessly vary the position of the second end of the connector on the intermediate blade.

The prosthetic implant also comprises an adjustment element that is activable on the slide to adjust the position of the slide on the intermediate blade. In this situation, varying the position of the second end of the connector makes it possible to vary the point where the force is applied. This also changes the lever arm of the force relative to the centre of rotation of the ankle (articulated joint), thus varying the transmitted torque, hence the torsional stiffness of the entire foot.

The technical purpose and the aims specified are also achieved by a method for adjusting a prosthetic implant of the ESR (energy-storing-and-returning) type. The prosthetic implant comprises an upper assembly configured to be connected to a patient's lower limb, and a lower assembly configured to define a foot having a front region, a middle region and a rear region. The lower assembly includes a lower blade, extending along a main direction between a heel end and a toe end.

The lower assembly includes an intermediate blade that is partly superposed on the lower blade, preferably in the middle region of the foot. The lower assembly includes an upper blade that is partly superposed on the intermediate blade, preferably in the middle region of the foot.

The blades are laminated springs made from composite material.

The upper, lower and intermediate blades are fastened stably to each other at at least one connecting point located in the front region or in the middle region of the foot and diverge from each other away from the connecting point towards the rear region of the foot.

The prosthetic implant also comprises an articulated joint - for example, a ball and socket joint, a rotoidal joint or a universal joint - interposed between the lower assembly and the upper assembly to allow relative motion between the lower assembly and the upper assembly.

The prosthetic implant also comprises a connector having a first end that is connected to the upper assembly, and a second end that is connected to the intermediate blade of the lower assembly to apply a force on the intermediate blade of the lower assembly.

More in detail, the connector acts as a rigid tendon element capable of transmitting a force between the upper assembly and the lower assembly. The method comprises a step of moving at least one between the first and the second end of the connector to vary the force applied by the connector on the intermediate blade of the lower assembly.

In the preferred embodiment, in the step of moving, it is the first end that moves, while the second end is articulated to the intermediate blade - hinged to it, for example.

In an embodiment, the upper assembly extends along a longitudinal direction between a hook-up end, where it is operatively connected to the patient's lower limb, and a fixing end, where it is fixed to the articulated joint. In this situation, the method comprises a step of sliding the first end of the connector relative to the upper assembly. In this situation, the movement direction is transverse, specifically perpendicular, to the longitudinal axis. In other words, to vary the force applied by the connector on the intermediate blade of the lower assembly, the first end of the connector is moved slidably along the movement direction.

The prosthetic implant also comprises a slider, to which the first end of the connector is pivoted. In this situation, the method also comprises a step of extracting or retracting the slider relative to the upper assembly along the movement direction through a plurality of equilibrium positions. The method also comprises a step of adjusting the position of the first end of the connector between a most extracted position, where the first end is distal to the upper assembly, and a least extracted position, where the first end is proximal to the upper assembly.

In other words, by extracting or retracting the slider relative to the upper assembly, it is possible to change in a controlled manner the position of the first end of the slider, where the force is exchanged between the foot and the upper assembly. Changing the position of the first end of the slider, that is, the point where the force is applied, changes the lever arm of the force relative to the centre of rotation of the ankle, hence the transmitted torque. In this situation, the blades change their point of rotation with the changing of the position of the first end of the connector, thus obtaining a non-linear variation of the torsional stiffness of the entire foot, capable of faithfully replicating the behaviour of a foot when subjected to stresses due to different loads and/or different ground conditions.

Further features and advantages of this invention are more apparent in the exemplary, hence non-limiting, description of an embodiment of a prosthetic implant of the ESR (energy-storing-and-returning) type and a method for adjusting a prosthetic implant of the ESR type.

### Brief description of the drawings

The description is set out below with reference to the accompanying drawings which are provided solely for purposes of illustration without limiting the scope of the invention and in which:
- Figure 1 shows a perspective view of the prosthetic implant;
- Figure 1A shows a detail from Figure 1;
- Figures 2A-2C show a side view of the prosthetic implant in three different configurations;
- Figure 3 shows a perspective view of the prosthetic implant of Figure 1, with an actuator applied to it;
- Figure 4 is a side view of a further embodiment of a prosthetic implant;
- Figure 4A shows a detail from Figure 4.

### Detailed description of preferred embodiments of the invention

With reference to the accompanying drawings, the numeral 100 denotes a prosthetic implant of the ESR (energy storing and returning) type.

The prosthetic implant 100 comprises an upper assembly 200 configured to be connected to a patient's lower limb.

The upper assembly 200 extends along a longitudinal direction A between a hook-up end 200a, where it is operatively connected to the patient's lower limb, and a fixing end 200b, where it is fixed to an articulated joint 500. The prosthetic implant 100 also comprises a lower assembly 300, configured to define a foot having a front region 300a, a middle region 300b and a rear region 300c.

The lower assembly 300 includes a lower blade 400a, extending along a main direction M, between a heel end 400a' and a toe end 400a".

In use, the main direction M coincides with the direction of forward movement of the patient while walking.

According to an aspect of this disclosure, the lower blade 400a has a curved shape along the main direction M, where the heel end 400a' and the toe end 400a" rest on the ground, while the part of the lower blade 400a between them is at least partly raised off the ground.

In other words, the lower blade 400a simulates the curvature and shape of the sole of a foot.

According to another aspect of this disclosure, the lower blade 400a has a slot I extending longitudinally on the lower blade 400a. In this situation, in the proximity of the toe end 400a", the slot I divides the lower blade 400a into two rounded portions L.

The lower assembly 300 also comprises an intermediate blade 400b, partly superposed on the lower blade 400a.

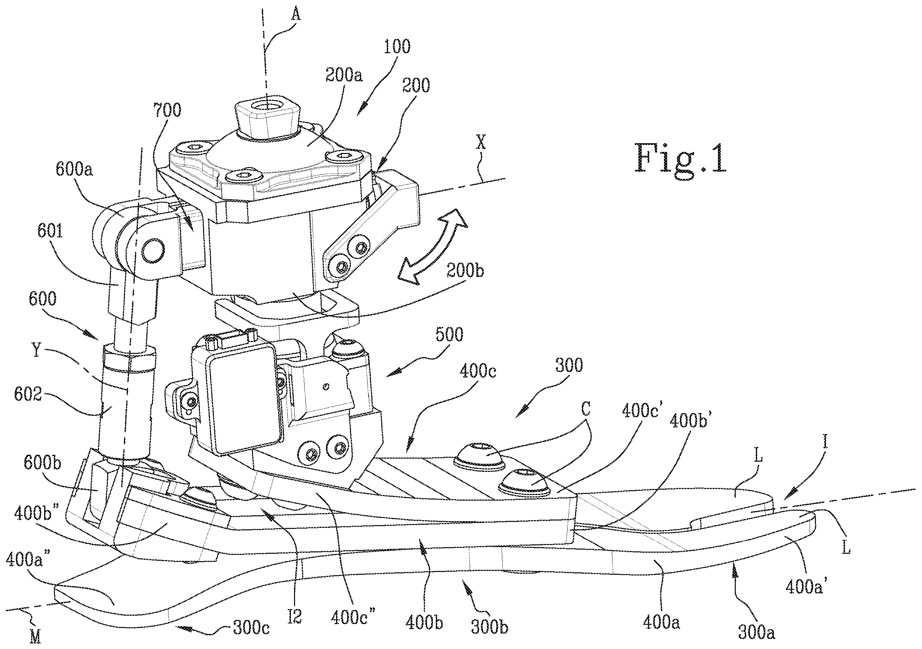

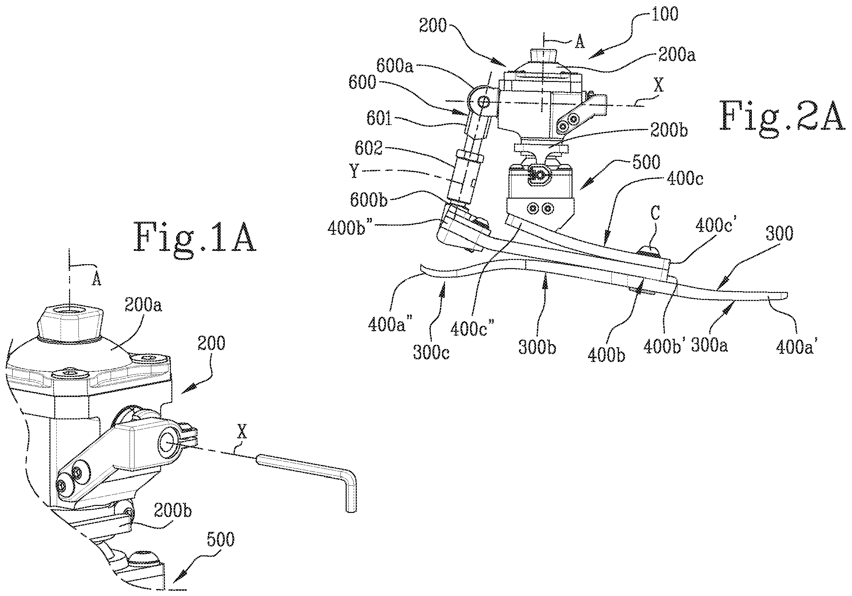

More specifically, as shown, for example, in Figure 1, the intermediate blade 400b is superposed on the lower blade 400a in proximity to the middle region 300b of the foot.

The intermediate blade 400b has a first, substantially rectilinear stretch extending from a toe end 400b' and a second stretch, lying at an angle to the first stretch and extending between the first stretch and a heel end 400b".

The intermediate blade 400b too is provided with a slot I2 extending longitudinally between the middle region 300b of the foot and the heel end 400b" of the intermediate blade 400b.

The lower assembly 300 also comprises an upper blade 400c, partly superposed on the intermediate blade 400b.

More specifically, as shown, for example, in Figure 1, the upper blade 400c is superposed on the intermediate blade 400b in proximity to the middle region 300b of the foot.

The upper blade 400c has a slight curvature extending from a toe end 400c' to a heel end 400c".

The upper blade 400c is shorter in length than the intermediate blade 400b. The intermediate blade 400b is shorter in length than the lower blade 400a. The upper, lower and intermediate blades 400a, 400b, 400c are fastened stably to each other at at least one connecting point C located in the front region 300a or in the middle region 300b of the foot and diverge from each other away from the connecting point C towards the rear region 300c of the foot.

In the embodiment shown in the accompanying drawings, the connecting point C is located in the middle region 300b of the foot.

In a possible embodiment, the connecting point C is embodied by two parallel pins extending between the upper blade 400c and the lower blade 400a.

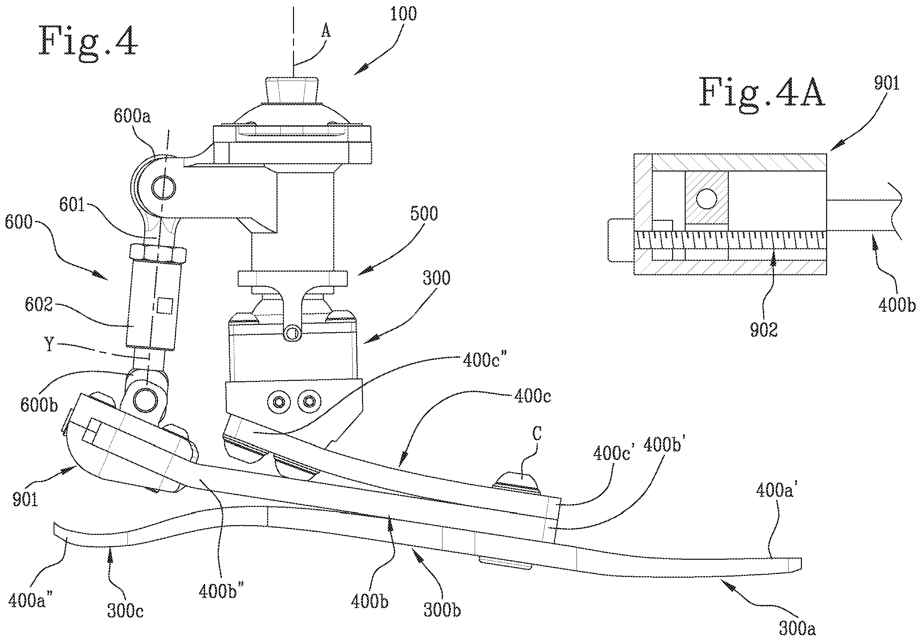

Aa shown, for example, in Figures 1 and 4, the upper blade 400c and the intermediate blade 400b are connected at the connecting point C in proximity to their respective toe ends 400b', 400c'.

The lower blade 400a, on the other hand, is connected to the other blades 400b, 400c in proximity to the middle region 300b of the foot.

According to an aspect of this disclosure, the lower blade 400c, the intermediate blade 400b and the upper blade 400a are laminated springs made from composite material.

When subjected to stress, the lower blade 400a, the intermediate blade 400b and the upper blade 400c have a non-linear elastic behaviour.

The prosthetic implant 100 also comprises an articulated joint 500, interposed between the lower assembly 300 and the upper one 200 to allow relative motion between the lower assembly 300 and the upper one 200.

In a possible embodiment, the articulated joint 500 includes a ball that is stably fixed to the upper assembly 200 and faces towards the lower assembly 300.

The articulated joint 500 also includes a receiving body that is fixed to the lower assembly 300 and faces towards the upper assembly 200 to receive the ball. In this situation, the ball and the receiving body define a ball and socket joint.

In the embodiment shown in the accompanying drawings, the receiving body is stably fixed to the upper blade 400c, preferably in the rear region 300c of the foot and in proximity to the heel end 400c" of the upper blade 400c itself.

The ball and socket joint 500 allows the upper assembly 200 and the lower assembly 300 to move relative to each other in that the ball can roll in the receiving body.

In other words, the ball and socket joint 500 simulates the patient's ankle articulation.

According to an aspect of this disclosure, the prosthetic implant 100 also comprises a connector 600 having a first end 600a that is connected to the upper assembly 200 and a second end 600b that is connected to the intermediate blade 400b of the lower assembly 300 to apply a force on the intermediate blade 400b of the lower assembly 300, as described in detail below. The connector 600 extends from the first end 600a to the second end 600b along a connection axis Y.

The connector 600 is located in proximity to the rear region 300c of the foot.

According to an aspect of this disclosure, one between the first and the second end 600a, 600b of the connector 600 is movable along a movement direction X to vary the force applied on the intermediate blade 400b of the lower assembly 300.

The movement direction X is inclined with respect to the connection axis Y. The movement direction X is inclined (preferably perpendicular) with respect to the longitudinal direction A. Thus, the displacement of the at least one between the first and the second end along the movement direction X causes the first or the second end of the connector 600 to move towards or away from the longitudinal axis A.

The connector 600 constrains the upper assembly 200 and the lower assembly 300 to rotate in a plane defined by the longitudinal axis A and by the movement direction, as shown by the arrow in Figure 1.

In other words, the connector 600 causes the articulated joint 500 to act as a centre of rotation for the prosthetic implant 100.

According to an aspect of this disclosure, the connector 600 is a rigid element.

According to another aspect of this disclosure, the connector 600 is adjustable (for example, it is variable in length).

Looking in more detail, the connector 600 comprises a first portion 601 extending away from the first end 600a, and a second portion 602 extending away from the second end 600b and engaged with the first portion 601. The first and the second portion 601, 602 are engaged with each other by a lead nut and screw coupling configured to set a length for using the connector 600 and to connect the first and the second portion 601, 602 rigidly so that the connector 600 acts as a rigid arm of preset length.

In this situation, by changing the position of one between the first and the second end 600a, 600b of the connector 600 it is possible to obtain, through the connector 600 itself, a variable lever arm so as to adapt the response of the prosthetic implant 100 as the patient moves.

In effect, by varying the lever arm, it is possible to change the point of rotation (or of flexion) of the blades 400a, 400b, 400c, varying their state of stress. Since the blades 400a, 400b, 400c are elastically deformable in non-linear manner, varying the lever arm makes it possible to vary the stiffness of the prosthetic implant 100 in non-linear manner so that the behaviour of the prosthetic implant 100 under stress is as close as possible to that of a natural foot.

In other words, changing the position of the point where the force is applied, changes the lever arm of the force relative to the centre of rotation of the articulated joint 500, hence the transmitted torque. The variation in the torsional stiffness obtained can be attributed to the entire foot, that is to the entire lower assembly 300.

In a possible embodiment shown in Figures 1, 2A-2C, 3, the first end 600a of the connector 600 is slidably movable relative to the upper assembly 200. In this situation, the movement direction X is transverse to the longitudinal axis A.

In the preferred embodiment, the movement direction X is perpendicular to the longitudinal direction A.

As shown in Figures 2A-2C, the prosthetic implant 100 comprises a slider 700 that is extractable or retractable relative to the upper assembly 200 along the movement direction X (that is, along a movement axis X defined by the slider 700) through a plurality of equilibrium positions.

Looking in more detail, the slider 700 is extracted slidably in a direction that is the opposite of the patient's direction of movement while walking, and is retracted slidably in the same direction as the patient's direction of movement while walking.

The first end 600a of the connector 600 is pivoted to the slider 700 - for example, hinged to it - to adjust the position of the first end 600a of the connector 600.

In other words, the position of the slider 700 along the movement direction X is adjustable (by extracting or retracting the slider 700) to position the first end 600a of the connector 600 at a predetermined distance from the upper assembly 200.

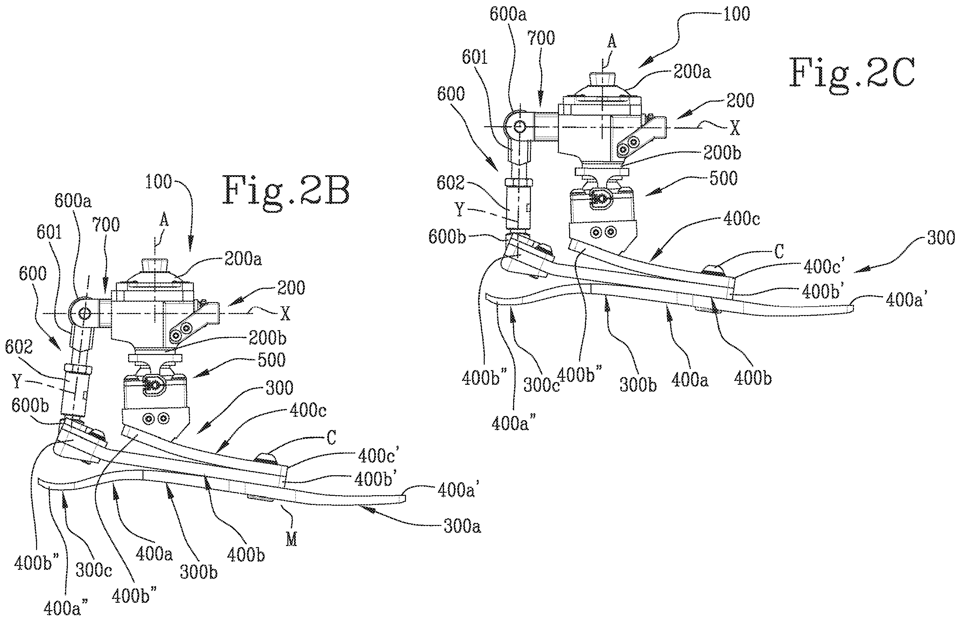

More specifically, the position of the first end 600a of the connector 600 can be adjusted between a most extracted position (Figure 2C), where the first end 600a is distal to the upper assembly 200, and a least extracted position (Figure 2A), where the first end 600a is proximal to the upper assembly 200. At the most extracted position, the prosthetic implant 100 exhibits a higher value of stiffness whilst, at the least extracted position, the prosthetic implant 100 exhibits a lower value of stiffness, as described in detail below. The first end 600a of the connector 600 is moved to the most extracted position when, for example, the patient lifts a load or walks on uneven ground and/or runs.

On the contrary, the first end 600a of the connector 600 is moved to the least extracted position when, for example, the patient needs to climb stairs or ramps or to walk on even ground.

Advantageously, the same prosthetic implant 100 is adaptable to the patient's different walking needs.

Also, the same prosthetic implant 100, being variable in stiffness, can be used by patients varying widely in body weight.

As shown in Figure 2B, the slider 700 can also be moved in such a way that the first end 600a of the connector 600 occupies an intermediate position between the most extracted and the least extracted position. At such an intermediate position, the prosthetic implant 100 is in a standard walking configuration, that is to say, under conditions allowing the patient to walk in what may be termed everyday life conditions.

According to an aspect of this disclosure, the slider 700 is coupled to the upper assembly 200 by a prismatic joint. In this situation, the slider 700 is made in the form of a prismatic section bar slidably extractable from, or insertable into, a housing made on the upper assembly 200 and shaped to match the bar.

Also, the second end 600b of the connector 600 is articulated to the intermediate blade 400b of the lower assembly 300.

In the embodiment of Figures 1, 2A-2C and 3, the second end 600b of the connector 600 is articulated to the intermediate blade 400b by a hinge.

In use, therefore, for the prosthetic implant 100 to be able to simulate a human foot as faithfully as possible, that is to say, to replicate the force-deformation curve representing the patient's natural gait cycle, the slider 700 is extracted from the upper assembly 200 by a predetermined quantity. In this situation, the connector 600 varies its inclination relative to the longitudinal direction A.

For example, if the slider 700 is moved to the most extracted position, the connecting point (identified with the first end 600a of the connector 600) between the slider 700 and the connector 600 occupies a position distal to the upper assembly 200 and the connector 600 is substantially parallel to the longitudinal direction A. On the contrary, if the slider 700 is moved to the least extracted position, the connecting point (that is, the first end 600a of the connector 600) between the slider 700 and the connector 600 occupies a position close to the upper assembly 200 and the connector 600 is substantially inclined with respect to the longitudinal direction A. According to an aspect of this disclosure, the change in position of the slider 700, hence of the first end 600a of the connector 600 along the movement direction X, is approximately 2 mm. In this situation, the stiffness of the prosthetic implant 100 changes by approximately 10 kg, allowing a good adjustment of its response to a change, for example, of the patient's weight. Since the blades 400a, 400b, 400c rest at least partly on each other and since they are laminated springs made from compositive material, their behaviour is a non-linear elastic behaviour. Changing the position of the first end 600a, that is, of the connecting point between the slider 700 and the connector 600, makes it possible to change the point of rotation of the blades 400a, 400b. 400c. In this situation, depending on the position of the first end 600a, the blades 400a, 400b, 400c are subjected to a different flexural state, thus allowing the elastic stiffness of the prosthetic implant 100 to be varied in non-linear manner.

Changing the position of the first end 600a of the connector 600 in a controlled manner varies the point where the force exchanged between the lower assembly 300 and the upper assembly 200 is applied. In this situation, the lever arm of the force relative to the centre of rotation of the ankle (that is, of the articulated joint 500) changes, hence the transmitted torque also changes.

In the embodiment of Figure 1, the position of the first end 600a of the connector 600, hence the extent to which the slider 700 is extracted, can be adjusted manually (Figure 1A).

In this situation, by using a adjustment tool, for example, an Allen key, it is possible to adjust the extent to which the slider 700 is extracted from the upper assembly 200, hence the position of the first end 600a of the connector 600 along the movement direction X.

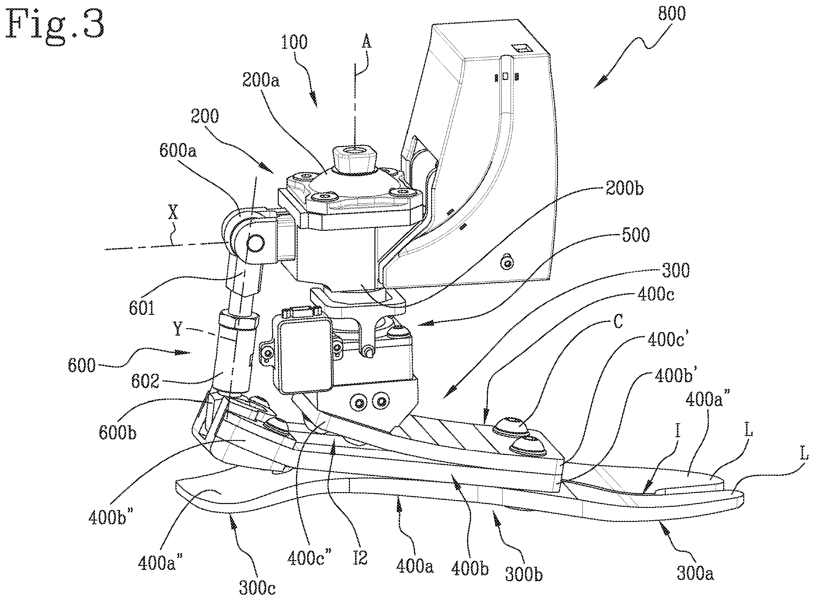

Alternatively, the prosthetic implant 100 comprises an actuator 800, shown in Figure 3, configured to slidably move the first end 600a of the connector 600.

In another embodiment, shown in Figures 4 and 4A, the end that is moved is the second end 600b of the connector 600.

In this situation, the second end 600b of the connector 600 is slidably movable along the intermediate blade 400b, while the first end 600a is hinged or pivoted to the upper assembly 200.

Looking in more detail, as shown in Figure 4A, the prosthetic implant 100 comprises a slide 901 that is slidably movable along the intermediate blade 400b to steplessly vary the position of the second end 600b of the connector 600 on the intermediate blade 400b.

The prosthetic implant 100 also comprises a locking element 902 that is applicable on the slide 901 to adjust the position of the slide 901 on the intermediate blade 400b.

According to an aspect of this disclosure, the locking element 902 can be applied to, and released from, the slide 901 to prevent or allow the slidable movement of the slide 901, respectively, so that its position along the intermediate blade 400b can be adjusted.

In the embodiment shown in the accompanying drawings, the locking element 902 is embodied in the form of a rotatable screw oriented substantially parallel to the intermediate blade 400b.

As shown in Figures 4 and 4A, the first end 600a of the connector 600 is pivoted to the upper assembly 200, while the second end 600b is engaged with the slide 901.

Adjusting the position of the second end 600b by sliding the slide 901 on the intermediate blade 400b makes it possible to vary the response of the prosthetic implant 100 so that it can adapt to different ground surface conditions in a manner almost identical to that of a natural foot.

In this situation, too, the force exchanged between the lower assembly 300 and the upper assembly 200 can be changed in a controlled manner by changing the position of the point where the force is applied, in this case the second end 600b of the connector 600. Changing the position of the point where the force is applied, changes the lever arm of the force relative to the centre of rotation of the ankle (that is, of the articulated joint 500), hence the transmitted torque. The variation in the torsional stiffness obtained can be attributed to the entire lower assembly 300.

Advantageously, the prosthetic implant 100 of this disclosure allows varying the stiffness of the lower assembly 300 non-linearly to adapt it to the patient's different walking needs.

More specifically, the lower assembly 300 constituting the foot is capable of faithfully replicating the force-deformation curve representing the behaviour of a natural foot when subjected to loads and/or when walking on uneven ground.

Another object of this disclosure is a method for adjusting a prosthetic implant 100 of the ESR (energy-storing-and-returning) type. The prosthetic implant 100 comprises an upper assembly 200 configured to be connected to a patient's lower limb, and a lower assembly 300 configured to define a foot having a front region 300a, a middle region 300b and a rear region 300c.

The lower assembly 300 includes a lower blade 400a extending along a main direction M, between a heel end 400a' and a toe end 400a"; an intermediate blade 400b partly superposed on the lower blade 400a, and an upper blade 400c partly superposed on the intermediate blade 400b.

The blades 400a, 400b, 400c are laminated springs made from composite material.

The blades 400a, 400b, 400c have a non-linear elastic behaviour.

The upper, lower and intermediate blades 400a, 400b, 400c are fastened stably to each other at at least one connecting point C located in the front region 300a or in the middle region 300b of the foot and diverge from each other away from the connecting point C towards the rear region 300c of the foot.

More specifically, the upper blade 400c and the intermediate blade 400b are connected at the connecting point C at a toe end 400b', 400c', while the lower blade 400a is connected at the connecting point C in proximity to the middle region 300b of the foot.

The prosthetic implant 100 also comprises an articulated joint 500, interposed between the lower assembly 300 and the upper assembly 200 to allow relative motion between the lower assembly 300 and the upper assembly 200.

More specifically, the articulated joint 500 simulates an ankle articulation.

The prosthetic implant 100 also comprises a connector 600 having a first end 600a that is connected to the upper assembly 200 and a second end 600b that is connected to the intermediate blade 400b of the lower assembly 300 to apply a force on the intermediate blade 400b of the lower assembly 300.

The connector 600 is rigid and preferably comprises a first and a second portion connected to each other by a lead nut and screw connection.

The method comprises a step of moving at least one between the first and the second end 600a, 600b of the connector 600 to vary the force applied by the connector 600 on the intermediate blade 400b of the lower assembly 300.

Changing in a controlled manner the position of one between the first and the second end 600a, 600b of the connector 600, where the force is exchanged between the lower assembly 300 and the upper assembly 200 means changing the point where the force is applied, thereby changing the lever arm of the force relative to the centre of rotation of the ankle (that is, relative to the articulated joint 500), hence the torque transmitted.

Since the blades 400a, 400b, 400c interact (by resting on each other), their points of rotation vary with the changing of the load applied to them. Changing the position of one of the ends 600a, 600b of the connector 600 varies the lever arm of the force and the points of rotation, and thus also the rigidity of the prosthetic implant 100 is varied in non-linear elastic manner. In this situation, the prosthetic implant 100 can be adapted to the user's different needs such as, for example, walking on uneven ground, carrying weights and the like.

According to an aspect of this disclosure, the upper assembly 200 extends along a longitudinal direction A between a hook-up end 200a, where it is operatively connected to the patient's lower limb, and a fixing end 200b, where it is fixed to the articulated joint 500. In this situation, the method comprises a step of sliding the first end 600a of the connector 600 relative to the upper assembly 200. In this situation, the movement direction X is transverse to the longitudinal axis A.

Thanks to the step of sliding, the position of the first end 600a of the connector 600 can be adjusted relative to the upper assembly 200 in such a way as to vary the stiffness of the entire prosthetic implant 100. According to an aspect of this disclosure, the prosthetic implant 100 comprises a slider 700 and the first end 600a of the connector 600 is pivoted to the slider 700.

**In** this situation, the method comprises a step of extracting or retracting the slider 700 relative to the upper assembly 200 along the movement direction X through a plurality of equilibrium positions, and a step of adjusting the position of the first end 600a of the connector 600 between a most extracted position, where the first end 600a is distal to the upper assembly 200, and a least extracted position, where the first end 600a is proximal to the upper assembly 200.

At the most extracted position, the stiffness of the prosthetic implant 100 is less than the stiffness when it is at the least extracted position.

Varying the position of the first end 600a of the connector 600by extracting or retracting the slider 700 varies the force exchanged between the lower assembly 300 and the upper assembly 200 via the slider 600. Varying the position of the first end 600a changes the point of application of the force, hence the lever arm of the force relative to the centre of rotation of the ankle (that is, relative to the articulated joint 500) is also varied, which in turn varies the torque transmitted.

In this situation, it is possible to adapt the foot to any type of ground and/or load which the patient needs to negotiate and/or support.

This invention achieves the preset aims and overcomes the disadvantages of the prior art.

In particular, the possibility of adjusting the position of one of the ends, specifically the first end, allows making the prosthetic implant adaptive, that is to say, it allows varying the stiffness of the prosthetic implant.

Moreover, the arrangement of the blades and their makeup allow varying the elastic stiffness of the prosthetic implant in non-linear manner, making the response of the prosthetic implant practically identical to the response of a foot of an able-bodied patient walking on different types of ground.

## Claims

1. A prosthetic implant (100) of the ESR (energy-storing-and-returning) type, comprising:
- an upper assembly (200) configured to be connected to a patient's lower limb;
- a lower assembly (300), configured to define a foot having a front region (300a), a middle region (300b) and a rear region (300c) and including:
a lower blade (400a), extending along a main direction (M), between a heel end (400a') and a toe end (400a");
an intermediate blade (400b), partly superposed on the lower blade (400a);
an upper blade (400c), partly superposed on the intermediate blade (400b);
wherein the upper, lower and intermediate blades (400a, 400b, 400c) are fastened stably to each other at at least one connecting point (C) located in the front region (300a) or in the middle region (300b) of the foot and diverge from each other away from the connecting point (C) towards the rear region (300c) of the foot;
- an articulated joint (500), interposed between the lower assembly (300) and the upper one (200) to allow relative motion between the lower assembly (300) and the upper one (300);
- a connector (600) having a first end (600a) that is connected to the upper assembly (200) and a second end (600b) that is connected to the intermediate blade (400b) of the lower assembly (300) to apply a force on the intermediate blade (400b) of the lower assembly (300),
**characterized in that** at least one of the first and the second end (600a, 600b) of the connector (600) is movable along a movement direction (X) to vary the force applied on the intermediate blade (400b) of the lower assembly (300).

2. The implant according to claim 1, wherein the upper assembly (200) extends along a longitudinal direction (A) between a hook-up end (200a), where it is operatively connected to the patient's lower limb, and a fixing end (200b), where it is fixed to the articulated joint (500), and wherein the first end (600a) of the connector (600) is slidably movable relative to the upper assembly (200), the movement direction (X) being transverse to the longitudinal axis (A).

3. The implant according to claim 2, comprising a slider (700), extractable or retractable relative to the upper assembly (200) along the movement direction (X) through a plurality of equilibrium positions, the first end (600a) of the connector (600) being pivoted to the slider (700) to adjust the position of the first end (600a) of the connector (600) between a most extracted position, where the first end (600a) is distal to the upper assembly (200), and a least extracted position, where the first end (600a) is proximal to the upper assembly (200).

4. The implant according to claim 3, wherein the slider (700) is coupled to the upper assembly (200) by a prismatic joint.

5. The implant according to any one of the preceding claims, wherein the second end (600b) of the connector (600) is articulated to the intermediate blade (400b) of the lower assembly (300), or wherein the implant comprises an actuator (800) configured to slidably move the first end (600a) of the connector (600).

6. The implant according to any one of the preceding claims, wherein the second end (600b) of the connector (600) is slidably movable along the intermediate blade (400b).

7. The implant according to claim 6, comprising:
- a slide (901) that is slidably movable along the intermediate blade (400b) to steplessly vary the position of the second end (600b) of the connector (600) on the intermediate blade (400b);
- an adjustment element (902) that is activable on the slide (901) to adjust the position of the slide (901) on the intermediate blade (400b).

8. The implant according to any one of the preceding claims, wherein the connector (600) is a rigid element and wherein the length of the connector (600) is adjustable.

9. The implant according to any one of the preceding claims, wherein the articulated joint (500) includes:
- a ball that is stably fixed to the upper assembly (200) and faces towards the lower assembly (300);
- a receiving body that is fixed to the lower assembly (300) and faces towards the upper assembly (200) to receive the ball;
the ball and the receiving body defining one between a ball and socket joint, a rotoidal joint or a universal joint.

10. The implant according to any one of the preceding claims, wherein the lower blade (400c), the intermediate blade (400b) and the upper blade (400a) are laminated springs made from composite material.

11. The implant according to any one of the preceding claims, wherein
- the connector (600) extends from the first end (600a) to the second end (600b) along a connection axis (Y);
- the movement direction (X) is inclined with respect to the connection axis (Y), or wherein
- the upper assembly (200) extends along a longitudinal direction (A) between a hook-up end (200a), where it is operatively connected to the patient's lower limb, and a fixing end (200b), where it is fixed to the articulated joint (500);
- the movement direction (X) is perpendicular to the longitudinal direction (A).

12. A method for adjusting a prosthetic implant (100) of the ESR (energy-storing-and-returning) type, according to claim 1, wherein the method comprises a step of moving at least one between the first and the second end (600a, 600b) of the connector (600) along a movement direction (X) to vary the force applied by the connector (600) on the intermediate blade (400b) of the lower assembly (300).

13. The method according to claim 12, wherein the upper assembly (200) extends along a longitudinal direction (A) between a hook-up end (200a), where it is operatively connected to the patient's lower limb, and a fixing end (200b), where it is fixed to the articulated joint (500), the method comprising a step of sliding the first end (600a) of the connector (600) relative to the upper assembly (200), the movement direction (X) being transverse to the longitudinal axis (A).

14. The method according to claim 13, wherein the implant comprises a slider (700) and wherein the first end (600a) of the connector (600) is pivoted to the slider (700), and wherein the method comprises the following steps:
- extracting or retracting the slider (700) relative to the upper assembly (200) along the movement direction (X) through a plurality of equilibrium positions;
- adjusting the position of the first end (600a) of the connector (600) between a most extracted position, where the first end (600a) is distal to the upper assembly (200), and a least extracted position, where the first end (600a) is proximal to the upper assembly (200).

15. The method according to any one of claims 12 to 14, wherein
- the connector (600) extends from the first end (600a) to the second end (600b) along a connection axis (Y);
- the movement direction (X) is inclined with respect to the connection axis (Y), or wherein
- the upper assembly (200) extends along a longitudinal direction (A) between a hook-up end (200a), where it is operatively connected to the patient's lower limb, and a fixing end (200b), where it is fixed to the articulated joint (500);
- the movement direction (X) is perpendicular to the longitudinal direction (A).

## Patentansprüche

1. Prothetisches Implantat (100) vom ESR-Typ (Energy Storage and Returning), umfassend:
- eine obere Baugruppe (200), die dazu ausgelegt ist, mit der unteren Extremität eines Patienten verbunden zu werden;
- eine untere Baugruppe (300), die dazu ausgelegt ist, einen Fuß zu definieren, der einen vorderen Bereich (300a), einen mittleren Bereich (300b) und einen hinteren Bereich (300c) aufweist und Folgendes einschließt:
eine untere Platte (400a), die sich entlang einer Hauptrichtung (M) zwischen einem Fersenende (400a') und einem Zehenende (400a") erstreckt;
eine Zwischenplatte (400b), die teilweise der unteren Platte (400a) überlagert ist;
eine obere Platte (400c), die teilweise der Zwischenplatte (400b) überlagert ist;
wobei die obere, untere und Zwischenplatte (400a, 400b, 400c) an mindestens einem Verbindungspunkt (C), der sich in dem vorderen Bereich (300a) oder in dem mittleren Bereich (300b) des Fußes befindet, stabil aneinander befestigt sind und voneinander wegführend von dem Verbindungspunkt (C) in Richtung des hinteren Bereichs (300c) des Fußes divergieren;
- eine Gelenkverbindung (500), die zwischen der unteren Baugruppe (300) und der oberen Baugruppe (200) angeordnet ist, um eine Relativbewegung zwischen der unteren Baugruppe (300) und der oberen Baugruppe (300) zu ermöglichen;
- einen Verbinder (600) mit einem ersten Ende (600a), das mit der oberen Baugruppe (200) verbunden ist, und einem zweiten Ende (600b), das mit der Zwischenplatte (400b) der unteren Baugruppe (300) verbunden ist, um eine Kraft auf die Zwischenplatte (400b) der unteren Baugruppe (300) auszuüben,
**dadurch gekennzeichnet, dass** mindestens das erste und/oder das zweite Ende (600a, 600b) des Verbinders (600) entlang einer Bewegungsrichtung (X) beweglich ist, um die auf die Zwischenplatte (400b) der unteren Baugruppe (300) ausgeübte Kraft zu variieren.

2. Implantat nach Anspruch 1, wobei sich die obere Baugruppe (200) entlang einer Längsrichtung (A) zwischen einem Einhackende (200a), an dem sie betriebswirksam mit der unteren Extremität des Patienten verbunden ist, und einem Befestigungsende (200b) erstreckt, an dem sie an der Gelenkverbindung (500) befestigt ist, und wobei das erste Ende (600a) des Verbinders (600) relativ zur oberen Baugruppe (200) verschiebbar beweglich ist, wobei die Bewegungsrichtung (X) quer zur Längsachse (A) ist.

3. Implantat nach Anspruch 2, umfassend einen Schieber (700), der relativ zur oberen Baugruppe (200) entlang der Bewegungsrichtung (X) durch eine Vielzahl von Gleichgewichtspositionen herausziehbar oder zurückziehbar ist, wobei das erste Ende (600a) des Verbinders (600) an dem Schieber (700) schwenkbar gelagert ist, um die Position des ersten Endes (600a) des Verbinders (600) zwischen einer am weitesten herausgezogenen Position, in der das erste Ende (600a) distal zur oberen Baugruppe (200) ist, und einer am wenigsten herausgezogenen Position, in der das erste Ende (600a) proximal zur oberen Baugruppe (200) ist, einzustellen.

4. Implantat nach Anspruch 3, wobei der Schieber (700) durch ein prismatisches Gelenk an die obere Baugruppe (200) gekoppelt ist.

5. Implantat nach einem der vorhergehenden Ansprüche, wobei das zweite Ende (600b) des Verbinders (600) an der Zwischenplatte (400b) der unteren Baugruppe (300) angelenkt ist, oder wobei das Implantat einen Aktuator (800) umfasst, der dazu ausgelegt ist, das erste Ende (600a) des Verbinders (600) verschiebbar zu bewegen.

6. Implantat nach einem der vorhergehenden Ansprüche, wobei das zweite Ende (600b) des Verbinders (600) entlang der Zwischenplatte (400b) verschiebbar beweglich ist.

7. Implantat nach Anspruch 6, umfassend:
- einen Schlitten (901), der entlang der Zwischenplatte (400b) verschiebbar beweglich ist, um die Position des zweiten Endes (600b) des Verbinders (600) auf der Zwischenplatte (400b) stufenlos zu variieren;
- ein Einstellelement (902), das an dem Schlitten (901) aktivierbar ist, um die Position des Schlittens (901) an der Zwischenplatte (400b) einzustellen.

8. Implantat nach einem der vorhergehenden Ansprüche, wobei der Verbinder (600) ein starres Element ist und wobei die Länge des Verbinders (600) einstellbar ist.

9. Implantat nach einem der vorhergehenden Ansprüche, wobei die Gelenkverbindung (500) Folgendes einschließt:
- eine Kugel, die stabil an der oberen Baugruppe (200) befestigt ist und der unteren Baugruppe (300) zugewandt ist;
- einen Aufnahmekörper, der an der unteren Baugruppe (300) befestigt ist und der oberen Baugruppe (200) zugewandt ist, um die Kugel aufzunehmen;
wobei die Kugel und der Aufnahmekörper eines zwischen einem Kugel- und Pfannengelenk, einem Rotoidengelenk oder einem Kardangelenk definieren.

10. Implantat nach einem der vorhergehenden Ansprüche, wobei die untere Platte (400c), die Zwischenplatte (400b) und die obere Platte (400a) laminierte Federn sind, die aus Verbundmaterial bestehen.

11. Implantat nach einem der vorhergehenden Ansprüche, wobei:
- sich der Verbinder (600) von dem ersten Ende (600a) zu dem zweiten Ende (600b) entlang einer Verbindungsachse (Y) erstreckt;
- die Bewegungsrichtung (X) gegenüber der Verbindungsachse (Y) geneigt ist, oder wobei
- sich die obere Baugruppe (200) entlang einer Längsrichtung (A) zwischen einem Einhackende (200a), an dem sie betriebswirksam mit der unteren Extremität des Patienten verbunden ist, und einem Befestigungsende (200b), an dem sie an der Gelenkverbindung (500) befestigt ist, erstreckt;
- die Bewegungsrichtung (X) senkrecht zur Längsrichtung (A) ist.

12. Verfahren zum Einstellen eines prothetischen Implantats (100) vom ESR-Typ (Energy Storage and Returning) nach Anspruch 1,
wobei das Verfahren einen Schritt zum Bewegen von mindestens dem ersten und/oder dem zweiten Ende (600a, 600b) des Verbinders (600) entlang einer Bewegungsrichtung (X) umfasst, um die von dem Verbinder (600) auf die Zwischenplatte (400b) der unteren Baugruppe (300) ausgeübte Kraft zu variieren.

13. Verfahren nach Anspruch 12, wobei sich die obere Baugruppe (200) entlang einer Längsrichtung (A) zwischen einem Einhackende (200a), an dem sie betriebswirksam mit der unteren Extremität des Patienten verbunden ist, und einem Befestigungsende (200b) erstreckt, an dem sie an der Gelenkverbindung (500) befestigt ist, wobei das Verfahren einen Schritt zum Verschieben des ersten Endes (600a) des Verbinders (600) relativ zur oberen Baugruppe (200) umfasst, wobei die Bewegungsrichtung (X) quer zur Längsachse (A) ist.

14. Verfahren nach Anspruch 13, wobei das Implantat einen Schieber (700) umfasst und wobei das erste Ende (600a) des Verbinders (600) an den Schieber (700) schwenkbar gelagert ist und wobei das Verfahren die folgenden Schritte umfasst:
- Herausziehen oder Zurückziehen des Schiebers (700) relativ zur oberen Baugruppe (200) entlang der Bewegungsrichtung (X) durch eine Vielzahl von Gleichgewichtspositionen;
- Einstellen der Position des ersten Endes (600a) des Verbinders (600) zwischen einer am weitesten herausgezogenen Position, in der das erste Ende (600a) distal zur oberen Baugruppe (200) ist, und einer am wenigsten herausgezogenen Position, in der das erste Ende (600a) proximal zur oberen Baugruppe (200) ist.

15. Verfahren nach einem der Ansprüche 12 bis 14, wobei
- sich der Verbinder (600) von dem ersten Ende (600a) zu dem zweiten Ende (600b) entlang einer Verbindungsachse (Y) erstreckt;
- die Bewegungsrichtung (X) gegenüber der Verbindungsachse (Y) geneigt ist, oder wobei
- sich die obere Baugruppe (200) entlang einer Längsrichtung (A) zwischen einem Einhackende (200a), an dem sie betriebswirksam mit der unteren Extremität des Patienten verbunden ist, und einem Befestigungsende (200b), an dem sie an der Gelenkverbindung (500) befestigt ist, erstreckt;
- die Bewegungsrichtung (X) senkrecht zur Längsrichtung (A) ist.

## Revendications

1. Implant prothétique (100) du type ESR (stockage et restitution d'énergie), comprenant :
- un ensemble supérieur (200) configuré pour être relié au membre inférieur d'un patient ;
- un ensemble inférieur (300), configuré pour définir un pied comportant une zone avant (300a), une zone centrale (300b) et une zone arrière (300c) et incluant :
une lame inférieure (400a), s'étendant le long d'une direction principale (M), entre une extrémité côté talon (400a') et une extrémité côté orteil (400a") ;
une lame intermédiaire (400b), partiellement superposée à la lame inférieure (400a) ;
une lame supérieure (400c), partiellement superposée à la lame intermédiaire (400b) ;
dans lequel les lames supérieure, inférieure et intermédiaire (400a, 400b, 400c) sont fixées de manière stable les unes aux autres à au moins un point de liaison (C) situé dans la zone avant (300a) ou dans la zone centrale (300b) du pied et divergent les unes des autres à partir du point de liaison (C) vers la zone arrière (300c) du pied ;
- un joint articulé (500), interposé entre l'ensemble inférieur (300) et l'ensemble supérieur (200) pour permettre un mouvement relatif entre l'ensemble inférieur (300) et l'ensemble supérieur (300) ;
- un connecteur (600) comportant une première extrémité (600a) qui est reliée à l'ensemble supérieur (200) et une seconde extrémité (600b) qui est reliée à la lame intermédiaire (400b) de l'ensemble inférieur (300) pour appliquer une force sur la lame intermédiaire (400b) de l'ensemble inférieur (300),
**caractérisé en ce qu'**au moins l'une des première et seconde extrémités (600a, 600b) du connecteur (600) est mobile le long d'une direction de mouvement (X) afin de faire varier la force appliquée sur la lame intermédiaire (400b) de l'ensemble inférieur (300).

2. Implant selon la revendication 1, dans lequel l'ensemble supérieur (200) s'étend le long d'une direction longitudinale (A) entre une extrémité d'accrochage (200a), où il est relié de manière fonctionnelle au membre inférieur du patient, et une extrémité de fixation (200b), où il est fixé au joint articulé (500), et dans lequel la première extrémité (600a) du connecteur (600) est mobile de manière coulissante par rapport à l'ensemble supérieur (200), la direction de mouvement (X) étant transversale à l'axe longitudinal (A).

3. Implant selon la revendication 2, comprenant un curseur (700), extractible ou rétractable par rapport à l'ensemble supérieur (200) le long de la direction de mouvement (X) à travers une pluralité de positions d'équilibre, la première extrémité (600a) du connecteur (600) étant pivotée sur le curseur (700) pour régler la position de la première extrémité (600a) du connecteur (600) entre une position la plus extraite, où la première extrémité (600a) est distale par rapport à l'ensemble supérieur (200), et une position la moins extraite, où la première extrémité (600a) est proximale par rapport à l'ensemble supérieur (200).

4. Implant selon la revendication 3, dans lequel le curseur (700) est couplé à l'ensemble supérieur (200) par une articulation prismatique.

5. Implant selon l'une quelconque des revendications précédentes, dans lequel la seconde extrémité (600b) du connecteur (600) est articulée à la lame intermédiaire (400b) de l'ensemble inférieur (300), ou dans lequel l'implant comprend un actionneur (800) configuré pour déplacer de manière coulissante la première extrémité (600a) du connecteur (600).

6. Implant selon l'une quelconque des revendications précédentes, dans lequel la seconde extrémité (600b) du connecteur (600) est mobile de manière coulissante le long de la lame intermédiaire (400b).

7. Implant selon la revendication 6, comprenant :
- un coulisseau (901) qui est mobile de manière coulissante le long de la lame intermédiaire (400b) pour faire varier en continu la position de la seconde extrémité (600b) du connecteur (600) sur la lame intermédiaire (400b) ;
- un élément de réglage (902) pouvant être actionné sur le coulisseau (901) pour régler la position du coulisseau (901) sur la lame intermédiaire (400b).

8. Implant selon l'une quelconque des revendications précédentes, dans lequel le connecteur (600) est un élément rigide et dans lequel la longueur du connecteur (600) est réglable.

9. Implant selon l'une quelconque des revendications précédentes, dans lequel le joint articulé (500) inclut :
- une bille qui est fixée de manière stable à l'ensemble supérieur (200) et qui est orientée vers l'ensemble inférieur (300) ;
- un corps de réception qui est fixé à l'ensemble inférieur (300) et qui est orienté vers l'ensemble supérieur (200) pour recevoir la bille ;
la bille et le corps de réception définissant l'un parmi un joint à rotule, un joint rotoïde ou un joint universel.

10. Implant selon l'une quelconque des revendications précédentes, dans lequel la lame inférieure (400c), la lame intermédiaire (400b) et la lame supérieure (400a) sont des ressorts laminés fabriqués à partir d'un matériau composite.

11. Implant selon l'une quelconque des revendications précédentes, dans lequel
- le connecteur (600) s'étend de la première extrémité (600a) à la seconde extrémité (600b) le long d'un axe de liaison (Y) ;
- la direction de mouvement (X) est inclinée par rapport à l'axe de liaison (Y), ou dans lequel
- l'ensemble supérieur (200) s'étend le long d'une direction longitudinale (A) entre une extrémité d'accrochage (200a), où il est relié de manière fonctionnelle au membre inférieur du patient, et une extrémité de fixation (200b), où il est fixé au joint articulé (500) ;
- la direction de mouvement (X) est perpendiculaire à la direction longitudinale (A).

12. Procédé de réglage d'un implant prothétique (100) du type ESR (stockage et restitution d'énergie) selon la revendication 1,
dans lequel le procédé comprend une étape consistant à déplacer au moins l'une des première et seconde extrémités (600a, 600b) du connecteur (600) le long d'une direction de mouvement (X) afin de faire varier la force appliquée par le connecteur (600) sur la lame intermédiaire (400b) de l'ensemble inférieur (300).

13. Procédé selon la revendication 12, dans lequel l'ensemble supérieur (200) s'étend le long d'une direction longitudinale (A) entre une extrémité d'accrochage (200a), où il est relié de manière fonctionnelle au membre inférieur du patient, et une extrémité de fixation (200b), où il est fixé au joint articulé (500), le procédé comprenant une étape consistant à faire coulisser la première extrémité (600a) du connecteur (600) par rapport à l'ensemble supérieur (200), la direction de mouvement (X) étant transversale à l'axe longitudinal (A).

14. Procédé selon la revendication 13, dans lequel l'implant comprend un curseur (700) et dans lequel la première extrémité (600a) du connecteur (600) est pivotée sur le curseur (700), et dans lequel le procédé comprend les étapes suivantes :
- extraire ou rétracter le curseur (700) par rapport à l'ensemble supérieur (200) le long de la direction de mouvement (X) à travers une pluralité de positions d'équilibre ;
- régler la position de la première extrémité (600a) du connecteur (600) entre une position la plus extraite, où la première extrémité (600a) est distale par rapport à l'ensemble supérieur (200), et une position la moins extraite, où la première extrémité (600a) est proximale par rapport à l'ensemble supérieur (200).

15. Procédé selon l'une quelconque des revendications 12 à 14, dans lequel
- le connecteur (600) s'étend de la première extrémité (600a) à la seconde extrémité (600b) le long d'un axe de liaison (Y) ;
- la direction de mouvement (X) est inclinée par rapport à l'axe de liaison (Y), ou dans lequel
- l'ensemble supérieur (200) s'étend le long d'une direction longitudinale (A) entre une extrémité d'accrochage (200a), où il est relié de manière fonctionnelle au membre inférieur du patient, et une extrémité de fixation (200b), où il est fixé au joint articulé (500) ;
- la direction de mouvement (X) est perpendiculaire à la direction longitudinale (A).
